(19) Europäisches Patentamt
European Patent Office
Office européen des brevets

(11) **EP 2 849 709 B1**

(12) **EUROPEAN PATENT SPECIFICATION**

(45) Date of publication and mention
of the grant of the patent:
**20.03.2024 Bulletin 2024/12**

(21) Application number: **13727433.8**

(22) Date of filing: **14.05.2013**

(51) International Patent Classification (IPC):
**A61F 13/514** *(2006.01)*

(52) Cooperative Patent Classification (CPC):
**A61F 13/51496**

(86) International application number:
**PCT/US2013/040836**

(87) International publication number:
**WO 2013/173261 (21.11.2013 Gazette 2013/47)**

(54) **ABSORBENT ARTICLES WITH UNIFORM GRAPHICS**

SAUGFÄHIGE ARTIKEL MIT GLEICHMÄSSIGER GRAFIK

ARTICLES ABSORBANTS DOTÉS DE GRAPHISMES UNIFORMES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **15.05.2012 US 201261646979 P**

(43) Date of publication of application:
**25.03.2015 Bulletin 2015/13**

(60) Divisional application:
**24154705.8 / 4 338 719**

(73) Proprietor: **The Procter & Gamble Company
Cincinnati, OH 45202 (US)**

(72) Inventors:
• **LAVON, Gary, Dean**
  **Cincinnati, OH 45202 (US)**
• **BICKING, Amanda, Margaret**
  **Cincinnati, OH 45202 (US)**
• **WALTHER, Rachel, Eden**
  **Cincinnati, OH 45202 (US)**
• **LU, Jonathan, Aaron**
  **Cincinnati, OH 45202 (US)**
• **TAPP, Ann, Cecilia**
  **Cincinnati, OH 45202 (US)**
• **YANG, Hui**
  **Cincinnati, OH 45202 (US)**
• **GLAHN, Tina, Marie**
  **Cincinnati, OH 45202 (US)**
• **WADE, Sarah, Marie**
  **Cincinnati, OH 45202 (US)**
• **JURATOVAC, Diana, W.**
  **Columbus, OH 43231 (US)**

(74) Representative: **P&G Patent Germany
Procter & Gamble Service GmbH
Sulzbacher Straße 40
65824 Schwalbach am Taunus (DE)**

(56) References cited:
EP-A1- 1 661 535          WO-A1-2011/090000
US-A1- 2004 243 083      US-A1- 2005 131 374
US-A1- 2006 264 858      US-A1- 2008 227 356
US-A1- 2011 092 942      US-A1- 2011 203 102
US-A1- 2011 288 517

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

FIELD OF THE INVENTION

The present invention relates to disposable pull-on garments.

BACKGROUND OF THE INVENTION

There is a desire to make disposable absorbent articles look more like underwear.. There are several factors that can affect whether an absorbent article is perceived as underwear-like. These factors include, but are not limited to, the noticeability of seams, the existence of graphics on a larger percentage of the viewable surfaces of the absorbent article, the appearance of waist and leg features and graphics flowing from or over two or more absorbent article components. WO2011/090000A1 discloses disposable wearing article comprising a graphic printed on a graphic film. US2006/0264858A1 discloses a training garment comprising first and second graphics printed on the outer surface of the training pant.

There are, however, many obstacles to designing and executing an absorbent article that is underwear-like. One is that absorbent articles are a compilation of separate article components and as new disposable absorbent article technologies are developed, they may result in the need for incorporation of additional, separate, article components. Because of the manner in which absorbent article components are incorporated, even the viewable surfaces of the article may have seams or areas of overlap or connection. Thus, it is challenging to place graphics on these individual components and to line them up such that the multi-component construction appears to be an integrated structure wherein the seams are de-emphasized and process variations are masked.

Further, depending on the type of article construction, it is challenging to maintain the uniform appearance of graphics that may be printed on more than one component. For example, a component with a particular graphic printed on it may eventually be incorporated into the article at a different depth than an adjacent component that also has a graphic printed on it. Depending on whether particular portions of a graphic are printed on an outermost layer, for example, or, if not, how opaque the layers may be through which the various portions of the graphic may be viewed, can have an impact on an entire article graphic that is meant to look uniform and consistent in that the various portions may have measureable differences in appearance when printed on different components or at different depths relative to the outer surface. For these and other reasons, it is challenging to create an absorbent article that comprises mainstream technologies and also that comprises graphics on multiple absorbent article components, such that the graphics flow from one absorbent article component to another article component in a manner that deemphasizes transitions from one component to another and creates a holistic underwear-like appearance.

It is a desire of the present invention to provide absorbent articles that look like underwear, while, at the same time, incorporating multiple absorbent article components that offer the benefits and functionality of the latest technologies (e.g., high stretch components integrated with low/no stretch components, highly breathable components, etc.). More specifically, it is a desire of the present invention to provide absorbent articles comprising graphics disposed on or spanning multiple viewable absorbent article components while creating a uniform appearance.

SUMMARY OF THE INVENTION

The present invention is for a pull-on disposable absorbent article as indicated in the appended claims.

BRIEF DESCRIPTION OF THE DRAWINGS

While the specification concludes with claims particularly pointing out and distinctly claiming the subject matter which is regarded as forming the present invention, it is believed that the invention will be better understood from the following description which is taken in conjunction with the accompanying drawings in which like designations are used to designate substantially identical elements, and in which:

> Figure 1 is a perspective view of an exemplary disposable pull-on garment in a typical in-use configuration;
> Figure 2 is a perspective view of an exemplary disposable pull-on garment in a typical in-use configuration;
> Figure 3 is a plan view of the pull-on garment in its flat uncontracted condition showing the inner surface;
> Figure 4a is a schematic cross section view of a first embodiment taken along line 4-4 in Figure 3 of an exemplary disposable pull-on garment;
> Figure 4b is a schematic cross section view of a second embodiment taken along line 4-4 in Figure 3 of an exemplary disposable pull-on garment;
> Figure 4c is a schematic cross section view of a third embodiment taken along line 4-4 in Figure 3 of an exemplary disposable pull-on garment;

Figure 4d is a schematic cross section view of a fourth embodiment taken along line 4-4 in Figure 3 of an exemplary disposable pull-on garment;

Figure 4e is a schematic cross section view of a sixth embodiment taken along line 4-4 in Figure 3 of an exemplary disposable pull-on garment;

Figure 4f is a schematic cross section view of a seventh embodiment taken along line 4-4 in Figure 3 of an exemplary disposable pull-on garment;

Figure 4g is a schematic cross section view of an eight embodiment taken along line 4-4 in Figure 3 of an exemplary disposable pull-on garment;

Figure 5 is a schematic cross section view taken along line 5-5 in Figure 3 of an example of a folded outer leg cuff suitable in one embodiment of the invention.

DETAILED DESCRIPTION OF THE INVENTION

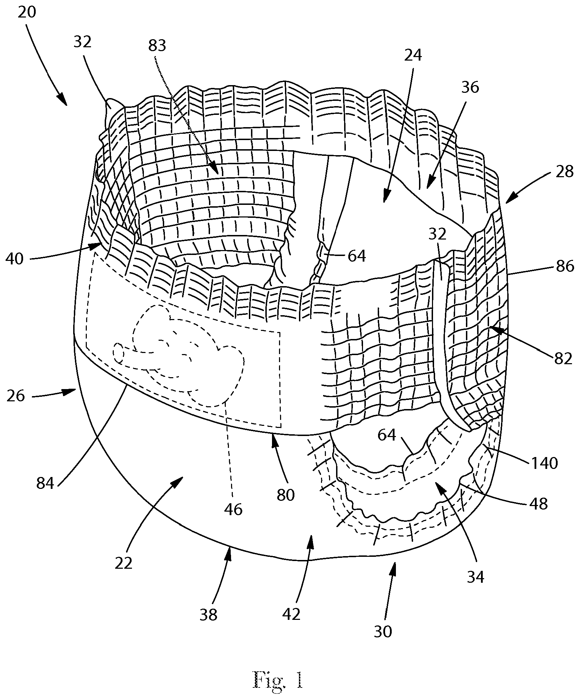

As used herein, the term "pull-on garment" refers to articles of wear which have a defined waist opening and a pair of leg openings and which are pulled onto the body of the wearer by inserting the legs into the leg openings and pulling the article up over the waist. The term "disposable" is used herein to describe garments which are not intended to be laundered or otherwise restored or reused as a garment (i.e., they are intended to be discarded after a single use and, preferably, to be recycled, composted or otherwise disposed of in an environmentally compatible manner). The pull-on garment is also preferably "absorbent" to absorb and contain the various exudates discharged from the body. A preferred embodiment of the absorbent article is the disposable absorbent pull-on garment, shown in Figure 1.

As used herein, the term "absorbent article" refers to pull-on garments generally worn by infants and other incontinent individuals to absorb and contain urine, feces and/or menses. It should be understood, however, that the term absorbent article is also applicable to other garments such as training pants, incontinent briefs, feminine hygiene garments or panties, and the like. In some embodiments, "absorbent article" may refer to a taped diaper.

As used herein, the terms "elastic," "elastomer," and "elastomeric" refer to a material which generally is able to extend to a strain of at least 50% without breaking or rupturing, and is able to recover substantially to its original dimensions after the deforming force has been removed.

The term "fibrous web" refers herein to a material made from continuous (long) filaments (fibers) and/or discontinuous (short) filaments (fibers) by processes such as spunbonding, meltblowing, carding, and the like. Examples include nonwovens, which do not have a defined woven or knitted filament pattern, and other textile coverstocks.

As used herein, the term "joined" encompasses configurations whereby an element is directly secured to another element by affixing the element directly to the other element, and configurations whereby an element is indirectly secured to another element by affixing the element to intermediate member(s), which in turn are affixed to the other element.

The term "substantial recovery" as used herein is intended to define the response of an elastic material wherein the material recovers at least 80% of the extension imported by the extension force and in most cases at least 90% of the extension. For example, a 4 inch (10.16 cm) sample extended 50% or two inches (5.08 cm) will recover at least 1.6 inches (4.064 cm) preferably at least 1.8 inches (4.572 cm) of the two inches (5.08 cm) of extension once the extension force is removed.

The term "substrate" is used herein to describe a material that is primarily two-dimensional (i.e., in an XY plane) and whose thickness (in a Z direction) is relatively small (i.e. 1/10 or less) in comparison to its length (in an X direction) and width (in a Y direction). Non-limiting examples of substrates include a web, layer or layers of fibrous materials, nonwovens, and films and foils, such as polymeric films or metallic foils, for example. These materials may be used alone or may comprise two or more layers laminated together. As such, a web may be a substrate or may be a laminate of two or more substrates.

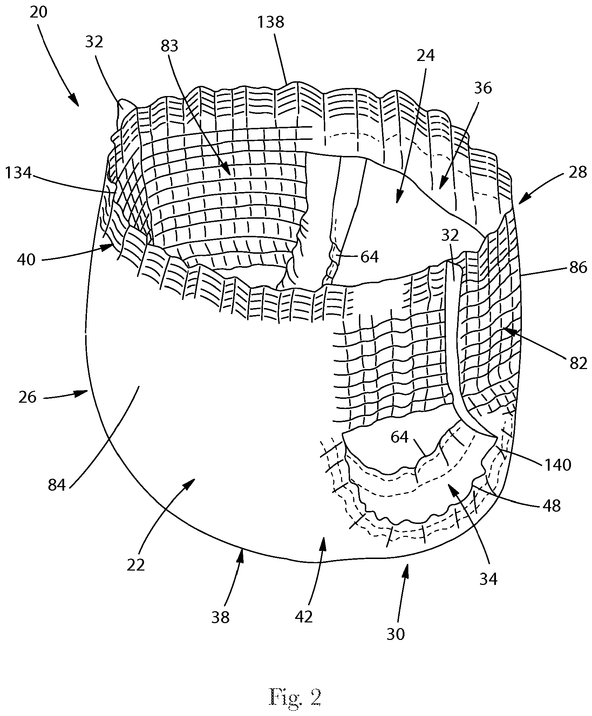

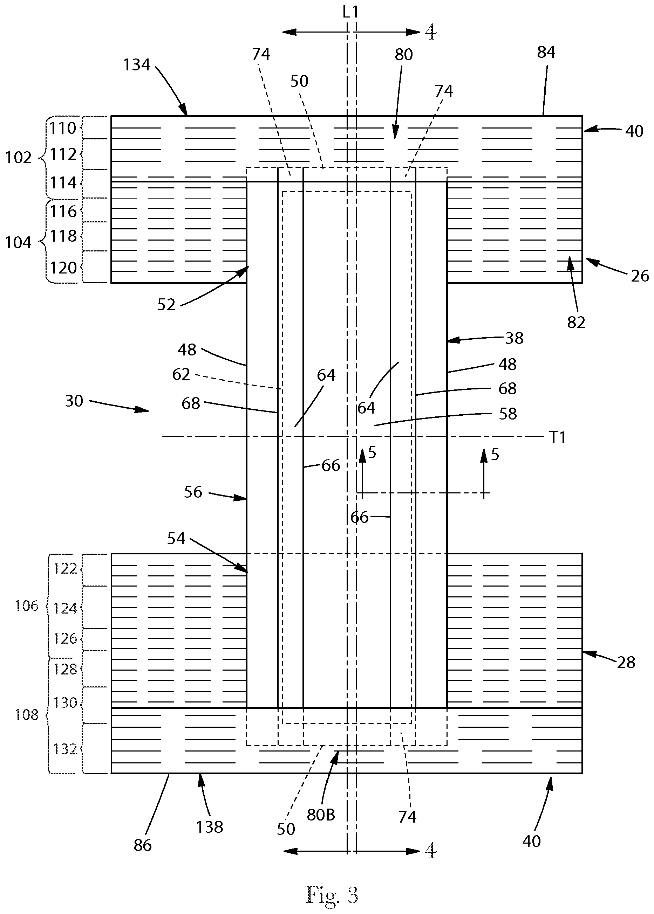

Figure 1 is a perspective view of the absorbent article 20. Figure 2 is a perspective view of the absorbent article 20. The absorbent article 20 has a longitudinal centerline L1 and a transverse centerline T1 (refer to Figure 3 as well). The absorbent article 20 has an outer surface 22, an inner surface 24 opposed to the outer surface 22, a front region 26, a back region 28, a crotch region 30, and seams 32 which join the front region 26 and the back region 28 to form two leg openings 34 and a waist opening 36.

In the embodiment shown in Figures 1, 2, and 3, the absorbent article 20 comprises an absorbent center chassis 38 to cover the crotch region of the wearer and a belt 40 extending transversely about the waist opening 36. The belt 40 includes a front elastomeric belt 84 and a rear elastomeric belt 86. The absorbent article 20 may also comprise an outer cover layer 42 to cover the center chassis 38. The belt 40 defines the waist opening 36. The belt 40, the center chassis 38 and/or the outer cover layer 42 may jointly define the leg opening 34.

In Figure 3, one or more of the belt layers or substrates may extend from a first waist edge 134 in a first waist region 26 through the crotch region to a longitudinally opposing second waist edge 138 in a second waist region 28 and forming a portion of the outer surface of the absorbent article 20.

The absorbent center chassis 38 absorbs and contains body exudates disposed on the center chassis 38. In the embodiment shown in Figure 3, the center chassis 38 has a generally rectangular shape having a longitudinal centerline L1, a transverse centerline T1, left and right longitudinally extending side edges 48 (hereinafter may be referred to as "longitudinal side edge") and front and back transversely extending end edges 50 (hereinafter may be referred to as "transverse end edge"). The center chassis 38 also has waist panels (i.e., a front waist panel 52 positioned in the front waist region 26 of the absorbent article 20 and a back waist panel 54 positioned in the back waist region 28) and a crotch panel 56 in the crotch region 30 between the front and back waist panels 52, 54. The center chassis and/or the crotch panel, that is, the portion of the center chassis that does not overlap either the front or rear elastomeric belts, may comprise at least one nonwoven layer and at least one film layer, each layer having an outer surface and an inner surface.

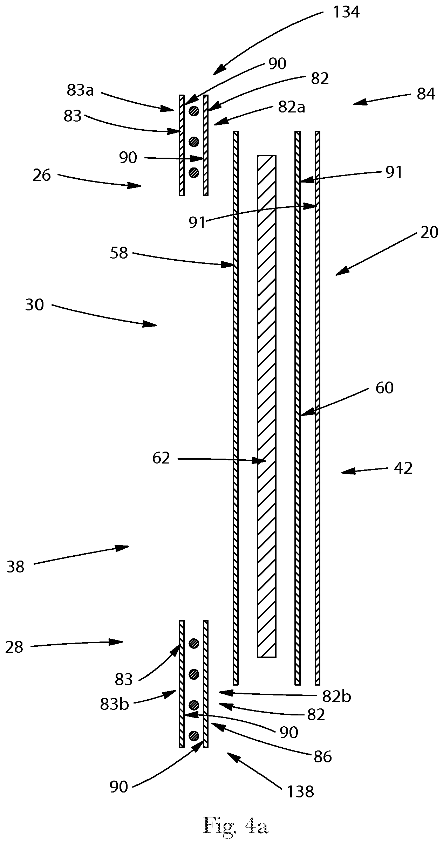

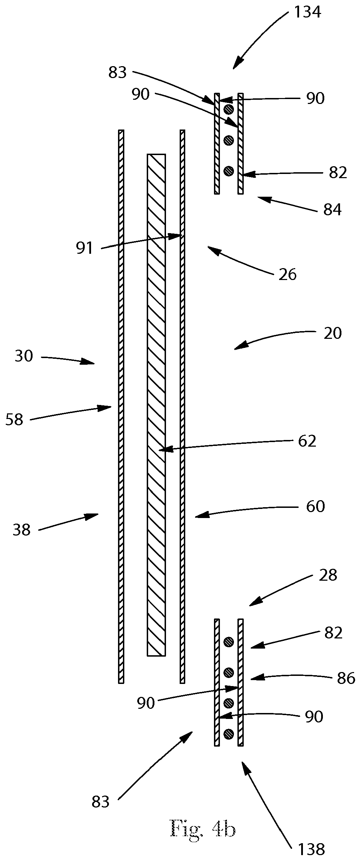

In the embodiment shown in Figure 4a and 4b, the absorbent articles 20 may comprise front and rear elastomeric belts 84, 86 intended to encircle at least a portion of the waist of the wearer, the front and rear belt portions 84, 86 being connected by a center chassis 38 forming the crotch region 30 of the absorbent article 20. The front and rear belts 84 and 86 may be formed from a first belt substrate 82 forming a portion of the outer surface 22(not shown) of the absorbent article, the first belt substrate may be formed of two longitudinally spaced webs of material. The front and rear belts 84 and 86 may also comprise a second belt substrate 83 forming a portion of the inner surface 24 (not shown) of the absorbent article 20, the second belt substrate 83 may also be formed of two longitudinally spaced webs of material. The second belt substrate may also be discontinuous and spaced apart in a transverse direction. The first and second belt substrates 82, 83 may be formed of substantially the same material or may comprise different materials. The first and second belt substrates 82, 83 may be formed from nonwovens, films, foams, elastic nonwoven, or combinations thereof. The front and rear belts 84, 86 may also comprise an elastomeric material disposed between the first and second belt substrates 82, 83. The elastomeric material may comprise one or more elastic strands, elastomeric films, elastomeric ribbons, elastomeric nonwovens, elastomeric filaments, elastomeric adhesives, elastomeric foams, scrims or combinations thereof. A portion of the elastomeric material may be directly combined with or form a portion of the outer cover layer or one or both of the belt substrates. The center chassis 38 of the absorbent article may comprise an outer surface 22, outer cover 42, an inner surface 24, topsheet 58, and an absorbent core 62 disposed between the topsheet 58 and the backsheet 60. The backsheet may be formed of any fibrous web, such as, for example, a nonwoven material, woven material, or films or laminates comprising a combination of one or more of these materials. The backsheet is a film and nonwoven laminate wherein the nonwoven of the laminate forms the outer cover layer. In addition, the center chassis 38 may comprise elasticized barrier leg cuffs 64 disposed at or adjacent the side edges of the center chassis. The front and rear belts 84, 86 may overlap at least a portion of the center chassis and one or both of the belt portions may be disposed on the outer surface of the center chassis or alternatively on the inner surface of the center chassis. A portion of the second belt substrate and/or a portion of the first belt substrate may be directly attached to the outer cover layer. Alternatively, the front belt and rear belt 84, 86 may comprise longitudinally spaced webs of material forming a first surface of the belt wherein the webs are folded along the waist edge, or alternatively the leg opening edge, of the belt to wrap the elastomeric material and form at least a portion of the second surface of the belt. In other words, at least a portion of the inner surface and outer surface of each of the belt portions may be formed from a single web of material.

The first belt substrate 82 may be disposed outward from the second belt substrate 83. Each of the first belt substrate and the second belt substrate comprises at least one nonwoven layer, each nonwoven layer having an outer surface and an inner surface, the outer surface disposed towards the outer surface of the absorbent article and the inner surface disposed towards the inner surface of the absorbent article. As shown in Figures 4a and 4b, the first belt substrate and/or the second belt substrate are in two discontinuous portions, a first portion in the front elastomeric belt 82a and 83a and a second portion in the rear elastomeric belt 82b and 83b.

A first graphic is printed on the outer surface of a nonwoven layer of the first and second portions of the first belt substrate. A second graphic is printed on the inner surface of the outer cover nonwoven that is in the crotch region, or the second graphic is printed on the outer surface of the film layer of the backsheet that is in the crotch region. In some embodiments, additional graphics may be printed on other layers of the article, for example, any of the layers of the belt substrates, the elastomeric material, the center chassis, or of the backsheet or outer cover. The number of nonwoven layers between any first graphic and the outer surface of the absorbent article is the same as the number of nonwoven layers between any second graphic and the outer surface of the article. In some embodiments, the number of nonwoven layers between a particular graphic and the outer surface of the article may be the same as the number of nonwoven layers between any or all other graphics and the outer surface of the article.

For example, in Figures 4a and 4b, a first graphic 90 may be printed on the outer surface of a nonwoven layer of the first and second portions of the second belt substrate. The first graphic may be viewed through one layer of nonwoven, as seen by looking at the outer surface of the article. A second graphic 91 may be printed on the inner surface of a nonwoven layer of the center chassis. Alternatively, a second graphic 91 may be printed on the outer surface of a center chassis film layer. In either case, the second graphic may also be viewed through one layer of nonwoven. Thus, the number of nonwoven layers between any first graphic and the outer surface of the absorbent article may be the same as the number of nonwoven layers between any second graphic and the outer surface of the article, resulting in a uniform appearance for all the graphics.

In some embodiments, there may be more than one nonwoven layer in either the first or second belt substrate. For example, in one embodiment, the first belt substrate may comprise two nonwoven layers, an inner nonwoven layer and an outer nonwoven layer. A first graphic may be printed on the outer surface of the inner nonwoven layer in both the first and second portions of the first belt substrate. A second graphic may be printed on either the inner surface of a center chassis nonwoven layer or the outer surface of a center chassis film layer. In such a case, the number of nonwoven layers between each of the first and second graphics and the outer surface of the article is the same, resulting in a uniform appearance for the article. The number of nonwoven layers between each of the first and second graphics and the outer surface of the article may be one, two, or any number, even zero if the graphics are printed on an outer surface of the absorbent article. What may maintain the uniform appearance of the total graphic over the entire article is that the number of nonwoven layers between each of the first and second graphics and the outer surface of the article is the same.

In the description for Figures 4a and 4b, as with the descriptions of other embodiments, it is understood that a nonwoven layer may have a measureable basis weight, such that a certain number of nonwoven layers may have substantially the same total basis weight as an equal number of other nonwoven layers. Furthermore, it should be understood that a certain number or nonwoven layers, one or more, may have substantially the same total basis weight as a different number of nonwoven layers. Similarly, the measureable opacity of a certain number of nonwoven layers may have substantially the same opacity as an equal number of other nonwoven layers. Furthermore, it should be understood that a certain number or nonwoven layers, one or more, may have substantially the same total opacity as a different number of nonwoven layers. Thus, when the number of nonwoven layers at a given basis weight is disposed between any first graphic and the outer surface of the absorbent article and the same number of nonwoven layers at the same basis weight is disposed between any second graphic and the outer surface of the article, the total basis weight of the materials between any first graphic and the outer surface of the absorbent article is substantially the same as the total basis weight of the materials between any second graphic and the outer surface of the article, thereby providing a uniform graphic appearance. Similarly, when the number of nonwoven layers between any first graphic and the outer surface of the article and the number of nonwoven layers between any second graphic and the outer surface of the article are different, it may be desirable that the basis weight of the layers between the first graphic and the outer surface and the second graphic and the outer surface are substantially the same such that the first graphic and second graphic provide a uniform graphic appearance as viewed from the outside of the article. Similarly, when the number of nonwoven layers between any first graphic and the outer surface of the absorbent article is different from the number of nonwoven layers between any second graphic and the outer surface of the article, it is desirable that the opacity of the materials between any first graphic and the outer surface of the absorbent article is substantially the same as the opacity of the materials between any second graphic and the outer surface of the article, such that the first graphic and second graphic provide a uniform graphic appearance. Thus a uniform graphic appearance may also be achieved by matching either the basis weight or the opacity of two components with graphics printed on them.

Additionally, other optical characteristics, such as gloss, laydown uniformity, and color, may also be similar between various regions comprising graphics printed on different surfaces and the outer surface of the absorbent articles, such that the overall graphic appears uniform. Thus, in some embodiments, the gloss of the nonwoven or aggregate nonwoven layers between any first graphic and the outer surface of the article may be the same as the gloss of the nonwoven or aggregate nonwoven layers between any second graphic and the outer surface of the article.

Examples of materials used in the present absorbent articles and the material basis weight and opacity may be an SMS web (spunbond-meltblown-spunbond web) with about 8 gsm basis weight (grams per square meter) and about 15% opacity (for example, as made by Avgol Nonwovens LTD, Tel Aviv, Israel, under the trade name XL-S70-26); an SMS web with about 11 gsm and about 18% to about 25% opacity; a softband SSS web with about 17 gsm and about 20% opacity (for example, as made by Pegas Nonwovens AS in Znojmo, Czech Republic, trade name 18 XX 01 00 01 00; XX = basis weight); an SSS web with about 15 gsm and about 18% opacity (for example, as made by Gulsan Sentetik Dok San VE TIC AS, in Gaziantep, Turkey, trade name SBXXF0YYY; XX=basis weight; YYY=slit width); an HESB (high extensibility spunbond) web with about 25 gsm and about 35% opacity (for example, as made by First Quality Nonwovens Inc., in Hazelton, Pennsylvania, trade name SEH2503XXX; XXX=slit width); and a bicomponent SS web with about 17 gsm and about 18% opacity. In some embodiments, the opacity of the nonwoven or aggregate nonwoven layers between any first graphic and the outer surface of the article may be from about 10% to about 50%, in some embodiments from about 5% to about 45%, in still other embodiments from about 10% to about 40%, or from about 10% to about 30%.

In some embodiments, the combined basis weight of the layers outward from the first graphic may be from about 8 gsm to about 38 gsm, in some embodiments from about 8 gsm to about 25 gsm, and in still other embodiments from about 6 gsm to about 35 gsm. In some embodiments, the basis weight (web) uniformity of the nonwoven or aggregate nonwoven layers may be less than about 20% the coefficient of variation, may be less than about 17.5%, or may be less than about 15%. Basis weight uniformity is measured by the coefficient of variation between basis weight taken for at least n=30 samples across a web, with a sample size that is 15mm x 25.4 mm.

In some embodiments, the gloss of a nonwoven or film material may be up to and including about 8 gloss units. In some embodiments, the gloss may be up to and including about 6 gloss units, in some embodiments, the gloss may be from about 4 to about 8 gloss units.

In some embodiments, such as in Figure 4b, the front and rear elastomeric belts may be disposed on the outer surface of the center chassis, in which case the second graphic may be printed on only the crotch panel portion of the center chassis, while the first graphic may be printed on the entire length of both portions of the first and second belt substrates, resulting in a substantially continuous graphic of uniform appearance from the first waist edge to the second waist edge. In some other embodiments, such as in Figure 4a, the front and rear elastomeric belts may be disposed on the inner surface of the center chassis, in which case the second graphic may be printed on the entire length of the center chassis, while the first graphic may be printed on only the portions of the first and second belt substrates that do not overlap the center chassis, again resulting in a substantially continuous graphic of uniform appearance from the first waist edge to the second waist edge.

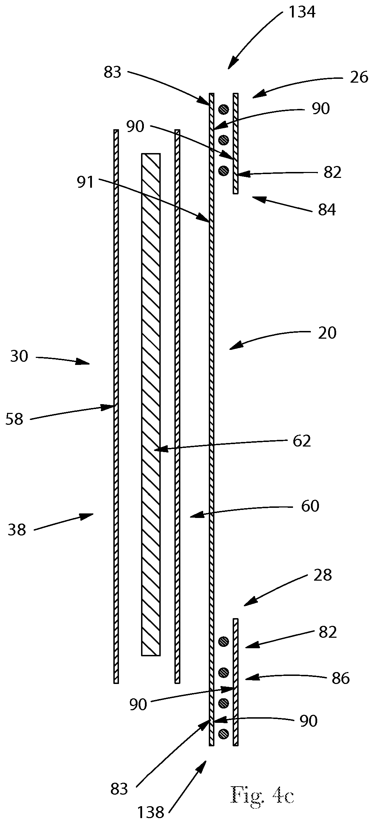

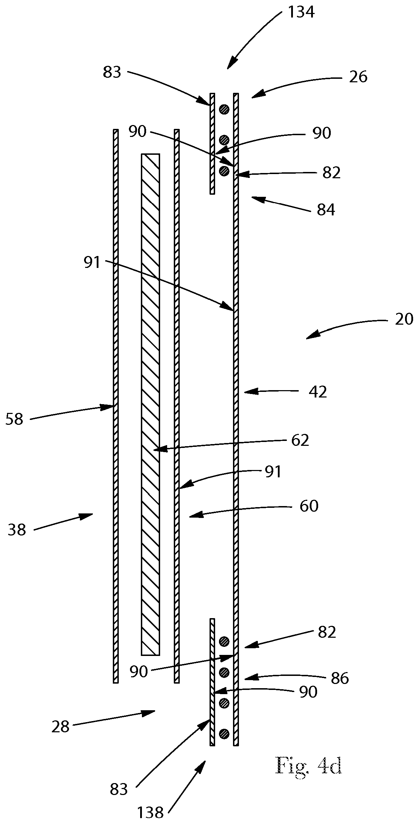

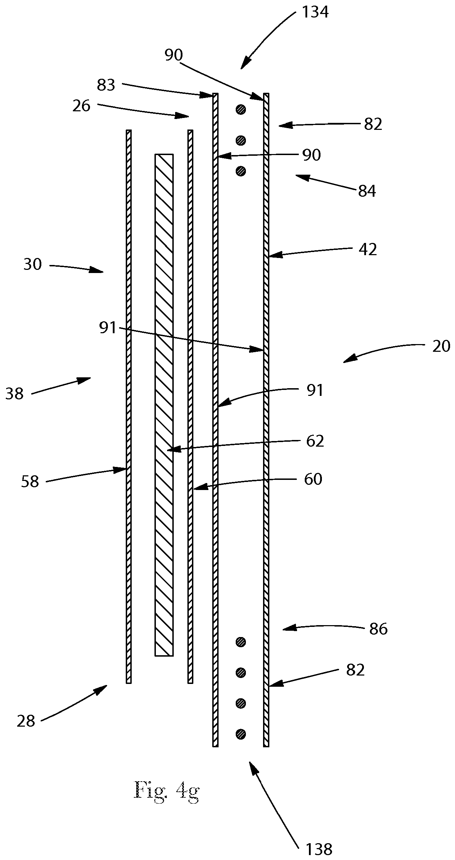

In the embodiments shown in Figure 4c, 4d, and 4g, the absorbent articles 20 may comprise front and rear elastomeric belts 84, 86 disposed in the front and rear waist regions 26, 28 respectively and intended to encircle at least a portion of the waist of the wearer, the front and rear belts 84, 86 being connected by the center chassis that forms the crotch region 30 of the article. The front and/or rear belt may be formed from the first and/or second belt substrate extending from a first waist edge 134 in a first waist region 26 through the crotch region to a longitudinally opposing second waist edge 138 in a second waist region 28 and forming a portion of the outer surface and/or inner surface of the absorbent article 20. The front and rear belts 84, 86 also may comprise a first and/or second belt substrate forming a portion of the inner surface 24 or outer surface 22, respectively, of the absorbent article, wherein the first and/or second belt substrate may be formed of two longitudinally spaced webs of material as shown in 4c and 4d. In such embodiments, the belts are formed by one continuous belt substrate extending from a first waist edge through the crotch to a second waist edge and the other belt substrate a pair of longitudinally spaced webs disposed in the longitudinally opposed waist regions.

In general, the first belt substrate 82 may be disposed outward from the second belt substrate 83. Each of the first belt substrate and the second belt substrate may have at least one nonwoven layer, each nonwoven layer having an outer surface and an inner surface, the outer surface disposed towards the outer surface of the absorbent article and the inner surface disposed towards the inner surface of the absorbent article. In some embodiments, the first belt substrate and/or the second belt substrate may be in two discontinuous portions, a first portion in the front elastomeric belt and a second portion in the rear elastomeric belt.

The front and rear belts may also comprise an elastomeric material disposed between the first and second belt substrates. The elastomeric material may comprise elastic strands, elastomeric films, elastomeric ribbons, elastomeric nonwovens, elastomeric filaments, elastomeric adhesives, elastomeric foams, scrims or combinations thereof. In some embodiments, a graphic may be printed or an outer surface of the elastomeric material or an inner surface of the elastomeric material, particularly if the elastomeric material is a film. The center chassis 38 of the absorbent article may comprise an outer surface 22, backsheet 60, an inner surface 24, topsheet 58, and an absorbent core 62 disposed between the topsheet 58 and the backsheet 60. The first belt substrate may form a portion of the outer surface 22. In addition, the center chassis may comprise elasticized barrier leg cuffs 64 disposed at or adjacent the side edges of the center chassis. The second belt substrate may overlap at least a portion of the center chassis and one or both of the second belt substrate webs may form the outer surface of the first belt or alternatively the inner surface of the first belt. Alternatively, the front portion and/or the rear portion of the first belt substrate may be folded along the waist edge of the belt region to wrap the elastomeric material and form a portion of the second belt substrate of one or both of the front and rear belt portions 84, 86. In other words, the inner surface and outer surface of each of the belt portions may be formed from a single web of material.

In Figure 4c, the first belt substrate may be in two discontinuous portions, a first portion in the front elastomeric belt and a second portion in the rear elastomeric belt. The second belt substrate may extend from a first waist edge in the first waist region through the crotch region to a longitudinally opposing second waist edge in the second waist region. A first graphic 90 may be printed on the outer surface of a nonwoven layer of the second belt substrate that is in the first and/or second waist regions. Alternatively, a first graphic may be printed on the inner surface of a nonwoven layer of the first and/or second portions of the first belt substrate. In either case, the first graphic may be viewed through one layer of nonwoven, as seen by looking at the outer surface of the article. A second graphic 91 may be printed on the inner surface of a nonwoven layer of the second belt substrate that is in the crotch region. The second graphic may also be viewed through one layer of nonwoven. Thus, the number of nonwoven layers between any first graphic and the outer surface of the absorbent article may be the same as the number of nonwoven layers between any second graphic and the outer surface of the article, resulting in a uniform appearance for all the graphics.

In Figure 4d, the first belt substrate may extend from a first waist edge in the first waist region through the crotch region to a longitudinally opposing second waist edge in the second waist region. The second belt substrate may be in two discontinuous portions, a first portion in the front elastomeric belt and a second portion in the rear elastomeric belt. A first graphic 90 may be printed on the outer surface of a nonwoven layer of the first and/or second portions of the second belt substrate. Alternatively, a first graphic 90 may be printed on the inner surface of a nonwoven layer of the first belt substrate that is in the first and/or second waist regions. In either case, the first graphic may be viewed through one layer of nonwoven, as seen by looking at the outer surface of the article. A second graphic 91 may be printed on the inner surface of a nonwoven layer of the first belt substrate that is in the crotch region. Alternatively, a second graphic 91 may be printed on the backsheet 60, that is, on the outer surface of a film layer of the backsheet. In either case, the second graphic may also be viewed through one layer of nonwoven. Thus, the number of nonwoven layers between any first graphic and the outer surface of the absorbent article may be the same as the number of nonwoven layers between any second graphic and the outer surface of the article.

In some embodiments, there may be more than one nonwoven layer in either the first or second belt substrate. For example, in one embodiment, the first belt substrate may comprise two nonwoven layers, an inner nonwoven layer and an outer nonwoven layer.

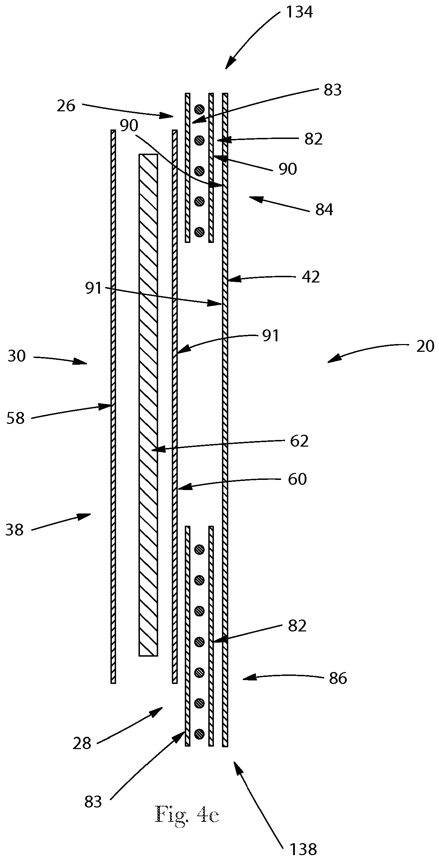

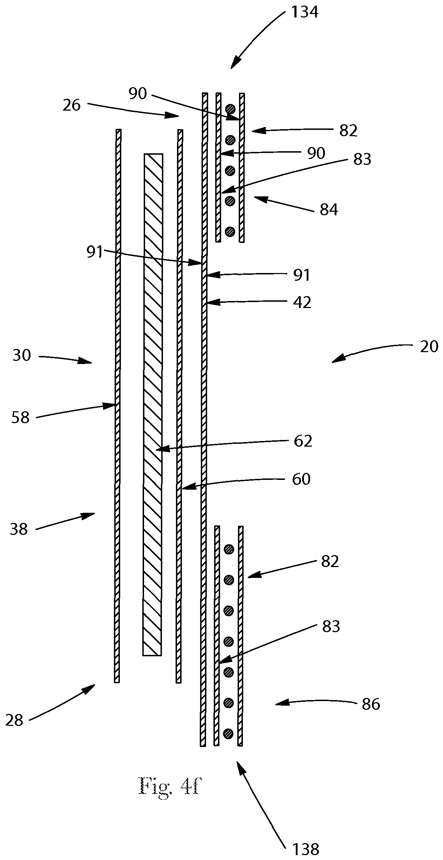

In the embodiments shown in Figure 4e and 4f, the absorbent article 20 may comprise a full outer cover layer 42, extending from a front waist edge 134 in a first waist region 26, through the crotch region to the longitudinally opposing rear waist edge 138 in a second waist region 28. The article may also comprise front and rear belts 84, 86 intended to encircle the waist of the wearer, the front and rear belts 84, 86 being connected to the outer cover layer 42 and/or the center chassis 38 of the absorbent article 20. The front and rear belts are formed from a first belt substrate forming a portion of the outer surface of the belt, the first belt substrate being formed of two longitudinally spaced webs of material. The front and rear belt portions also comprise a second belt substrate forming a portion of the inner surface of the absorbent article, the second belt substrate also being formed of two longitudinally spaced webs of material. The first and second belt substrates may be formed of substantially the same material or may comprise different materials. The first and second belt substrates may be formed from nonwovens, films, foams or combinations thereof. The front and rear belts may also comprise an elastomeric material disposed between the first and second belt substrates. The elastomeric material may comprise elastic strands, elastomeric films, elastomeric ribbons, elastomeric nonwovens, elastomeric filaments, elastomeric adhesives, elastomeric foams, scrims or combinations thereof.

The front and/or rear belts may be disposed on the interior surface of the outer cover layer, such as in Figure 4e. Alternatively, the front and/or rear belts may be disposed on the exterior surface of the outer cover layer, such as in Figure 4f. In one such embodiment the outer cover layer would form a portion of the inner surface of the article in the waist regions and the first belt substrate would form a portion of the outer surface of the article. The second belt substrate when present may be disposed between the first belt substrate and the outer cover layer. The center chassis 38 of the absorbent article 20 may comprise an outer surface 22, backsheet 60, an inner surface 24, topsheet 58, and an absorbent core 62 disposed between the topsheet 58 and the backsheet 60. In addition, the center chassis 38 may comprise elasticized barrier leg cuffs 64 disposed at or adjacent the side edges of the center chassis 38. One or both of the front and rear belts 84, 86 may overlap at least a portion of the center chassis 38 and one or both of the belts may be disposed on the outer surface of the center chassis 38 or alternatively on the inner surface of the center chassis 38. One or both of the front belt and rear belt 84, 86 may comprise longitudinally spaced webs of material forming a first surface of the belt wherein the webs are folded along the waist edge 36 of the belt to wrap the elastomeric material and form at least a portion of the second surface of the belt. In other words, a portion or the entirety of the inner surface and outer surface of one or both of the belt portions may be formed from a single web of material. The rugosities, wrinkles, folds in one or both of the front and rear belts may have a different configuration, size, orientation, shape, etc. than that of the outer cover layer. In the embodiment in which the outer cover layer 42 is disposed exteriorly of the front and/or rear belts, the outer cover layer may be folded along the waist edge to overlap the end of the front and/or rear belts and form a portion of the interior surface of the article.

In general, the first belt substrate may be disposed outward from the second belt substrate 83. Each of the first belt substrate and the second belt substrate may have at least one nonwoven layer, each nonwoven layer having an outer surface and an inner surface, the outer surface disposed towards the outer surface of the absorbent article and the inner surface disposed towards the inner surface of the absorbent article. The full outer cover may have an outer surface and an inner surface. In some embodiments, the first belt substrate and/or the second belt substrate may be in two discontinuous portions, a first portion in the front elastomeric belt and a second portion in the rear elastomeric belt.

In Figure 4e, a first graphic 90 may be printed on the inner surface of the outer cover that is in the first and second waist regions. Alternatively, a first graphic 90 may be printed on the outer surface of a nonwoven layer of the first and/or second portions of the first belt substrate. In either case, the first graphic may be viewed through one layer of nonwoven, as seen by looking at the outer surface of the article. A second graphic 91 may be printed on the outer surface of a film in the backsheet 60 that is in the crotch region. Alternatively, a second graphic 91 may be printed on the inner surface of the full outer cover that is in the crotch region. In either case, the second graphic may also be viewed through one layer of nonwoven. Thus, the number of nonwoven layers between any first graphic and the outer surface of the absorbent article may be the same as the number of nonwoven layers between any second graphic and the outer surface of the article, resulting in a uniform appearance for all the graphics.

In Figure 4f, a first graphic 90 may be printed on the inner surface of the first and/or second portions of the first belt substrate. Alternatively, a first graphic 90 may be printed on the outer surface of a nonwoven layer of the first and second portions of the second belt substrate. In either case, the first graphic may be viewed through one layer of nonwoven, as seen by looking at the outer surface of the article. A second graphic 91 may be printed on the inner surface of the full outer cover in the crotch region or on the outer surface of backsheet 60 in the crotch region. In either case, the second graphic may also be viewed through one layer of nonwoven. Thus, the number of nonwoven layers between any first graphic and the outer surface of the absorbent article may be the same as the number of nonwoven layers between any second graphic and the outer surface of the article, resulting in a uniform appearance for all the graphics.

In some embodiments, there may be more than one nonwoven layer in either the first or second belt substrate. For example, in one embodiment, the first belt substrate may comprise two nonwoven layers, an inner nonwoven layer and an outer nonwoven layer.

In the embodiment shown in Figure 4g, the absorbent articles 20 may comprise front and rear belts 84, 86 intended to encircle at least a portion of the waist of the wearer, the front and rear belts 84, 86 being connected to a center chassis 38 forming a portion of the crotch region 30 of the absorbent article 20. The front and rear belts 84, 86 comprise a first belt substrate extending from a first waist edge in a first waist region, through the crotch region to a second waist edge in a second waist region and forming a portion of the outer surface of the absorbent article. The front and rear belts 84, 86 also comprise a second belt substrate 83 extending from a first waist edge in a first waist region, through the crotch region to a second waist edge in a second waist region and forming a portion of the inner surface 24 of the absorbent article 20. In certain embodiments, the second belt substrate may be laterally discontinuous and spaced apart in a transverse direction. The first and second belt substrates 82, 83 may be formed of substantially the same material or may comprise different materials and may form a portion of the outer cover layer. The first and second belt substrates 82, 83 may be formed from nonwovens, films, foams or combinations thereof. The front and rear belt portions 84, 86 may also comprise an elastomeric material disposed between or forming a portion of one or both of the first and second belt substrates 82, 83. The elastomeric material may comprise elastic strands, elastomeric films, elastomeric ribbons, elastomeric nonwovens, elastomeric filaments, elastomeric adhesives, elastomeric foams, scrims or combinations thereof. A portion of the elastomeric material may be directly combined with the outer cover layer. The center chassis 38 of the absorbent article may comprise an outer surface 22, backsheet 60, an inner surface 24, topsheet 58, and an absorbent core 62 disposed between the topsheet 58 and the backsheet 60. In certain embodiments the backsheet may be a nonwoven and film laminate wherein the nonwoven is formed by the outer cover layer. In addition, the center chassis 38 may comprise elasticized barrier leg cuffs 64 disposed at or adjacent the side edges of the center chassis 38. The front and rear belts 84, 86 overlap at least a portion of the center chassis 38 and may be disposed on the outer surface of the center chassis 38. A portion of the second belt substrate and/or a portion of the first belt substrate may be directly attached to the outer cover layer. The front and rear belts 84, 86 may be formed from a first belt substrate extending from a first waist edge 134 in a first waist region 26 through the crotch region to a second waist edge 138 in a second waist region 28 and forming a portion of the outer surface of the absorbent article 20. The front and rear belts 84, 86 may also comprise a second belt substrate extending from a first waist edge 134 in a first waist region 26 through the crotch region to a second waist edge 138 in a second waist region 28 and forming a portion of the inner surface of the absorbent article 20.

In general, the first belt substrate may be disposed outward from the second belt substrate 83. Each of the first belt substrate and the second belt substrate may have at least one nonwoven layer, each nonwoven layer having an outer surface and an inner surface, the outer surface disposed towards the outer surface of the absorbent article and the inner surface disposed towards the inner surface of the absorbent article. The full outer cover may have an outer surface and an inner surface.

In Figure 4g, a first graphic 90 may be printed on the inner surface of a nonwoven layer of the first belt substrate that is in the first and second waist regions. Alternatively, a first graphic 90 may be printed on the outer surface of a nonwoven layer of the second belt substrate that is in the first and second waist regions. In either case, the first graphic may be viewed through one layer of nonwoven, as seen by looking at the outer surface of the article. A second graphic 91 may be printed on the inner surface of a nonwoven layer of the first belt substrate that is in the crotch region. Alternatively, a second graphic may be printed on the outer surface of a nonwoven layer of the second belt substrate that is in the crotch region. In either case, the second graphic may also be viewed through one layer of nonwoven. Thus, the number of nonwoven layers between any first graphic and the outer surface of the absorbent article may be the same as the number of nonwoven layers between any second graphic and the outer surface of the article, resulting in a uniform appearance for all the graphics.

In some embodiments, there may be more than one nonwoven layer in either the first or second belt substrate. For example, in one embodiment, the first belt substrate may comprise two nonwoven layers, an inner nonwoven layer and an outer nonwoven layer.

A portion or the whole of the center chassis 38 may be made extensible to a degree greater than the inherent extensibility of the material or materials from which the center chassis 38 is made, *e.g.*, the backsheet 60. The additional extensibility may be desirable in order to allow the center chassis 38 to conform to the body of a wearer during movement by the wearer and or to provide adequate body coverage. The additional extensibility may also be desirable, for example, in order to allow the user of a absorbent article including a center chassis 38 having a particular size before extension to extend the front waist region 26, the back waist region 28, or both waist regions of the center chassis 38 to provide additional body coverage for wearers of differing size, *i.e.*, to tailor the article to the individual wearer. Such extension of the waist region or regions may give the center chassis 38 a generally hourglass shape, so long as the crotch region is extended to a relatively lesser degree than the waist region or regions, and may impart a tailored appearance to the article when it is worn. In addition, the additional extensibility may be desirable in order to minimize the cost of the article. For example, an amount of material that would otherwise be sufficient only to make a relatively smaller article lacking this extensibility can be used to make an article capable of being extended to adequately cover a wearer that is larger than the unextended smaller absorbent article would fit.

A portion of the center chassis 38, for example a portion of the chassis in one or both of the waist regions 26, 28 may be made laterally extensible to a maximum extensibility greater than a maximum extensibility of another portion of the center chassis 38 in the crotch region such that a lateral extension of each of the portions to its maximum extensibility imparts an hourglass shape to the center chassis 38. In one embodiment, the portion of the center chassis 38 underlying and/or immediately adjacent one or both of the front and back extensible belts may be made laterally extensible to a maximum extensibility greater than a maximum extensibility of another portion of the center chassis 38, for example the crotch region, such that a lateral extension of each of the portions to its maximum extensibility facilitates application of the absorbent article onto the body of a wearer by enabling the waist regions to be extended to fit over the wearer's hips and in addition, opening and orienting the leg openings enabling the wearer to place the legs through the openings more effectively.

Additional lateral extensibility in the center chassis 38 may be provided in a variety of ways. For example, a material or materials from which the center chassis 38 is made may be pleated by any of many known methods. Alternatively, all or a portion of the center chassis 38 may be made of a formed web material or a formed laminate of web materials like those described in U.S. Patent No. 5,518,801 issued on 21 May 1996 in the name of Chappell et al. This formed web material includes distinct laterally extending regions in which the original material has been altered by embossing or another method of deformation to create a pattern of generally longitudinally oriented alternating ridges and valleys and also includes laterally extending unaltered regions between the laterally extending altered regions. The formed web material can be extended in a direction perpendicular to the ridges up to the point where the ridges and valleys flatten with substantially less force than is required to extend beyond that point. In addition to lateral extensibility, the creation of a formed laminate web as described above provides a center chassis 38 backsheet with improved texture and cloth-like appearance and feel. The deformation creates a cloth-like pattern in the film and increases the loft of the nonwoven in multi-layer film and nonwoven laminate backsheets.

Alternatively, a portion of the absorbent article can be ring-rolled and thus rendered highly extensible as described in US5,366,782 (issued Nov. 22, 1994 to Curro, et al). Specifically, a ring-rolling apparatus includes opposing rolls having intermeshing teeth that incrementally stretch and thereby plastically deform the material forming the absorbent article (or a portion thereof) thereby rendering the article extensible in the ring-rolled regions. In one embodiment, the absorbent article can be ring-rolled in a portion of at least one of the front or back waist regions, for example the portion of the center chassis 38 underlying and/or immediately adjacent one or both of the front and back belts 84, 86, while other regions may comprise a structured elastic-like formed web material. The article may be ring-rolled across the entire width in one or both of the waist regions or alternatively may be ring-rolled over only a portion of the center chassis 38 width.

The front laterally central portion and the back laterally central portion of the center chassis 38 may have a different range of extensibility from other portions of the center chassis 38. Additionally or alternatively, the laterally central portions may be extensible to a greater or lesser degree when subjected to a given level of opposing tensile forces, *i.e.*, may be more easily or less easily extensible, than other portions of the center chassis 38.

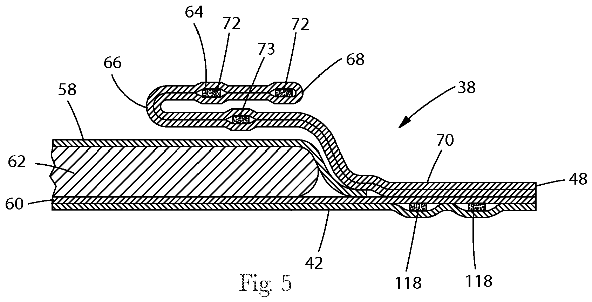

The center chassis 38 may comprise a liquid pervious topsheet 58, a liquid impervious backsheet 60 and an absorbent core 62 disposed there between. The center chassis 38 may additionally comprise a barrier leg cuff 64 disposed along the longitudinal side edge 48. The barrier leg cuff 64 provides improved containment of liquids and other body exudates in the crotch region 30. The barrier leg cuff 64 shown in Figure 5 comprises a single layer of material which may be folded to form a barrier leg cuff having two layers. The barrier leg cuff 64 extends from the side of the center chassis at or adjacent the longitudinal side edge 48 toward the longitudinal centerline L1. The barrier leg cuff may be folded along the folding line 66 back toward the longitudinal side edge 48. The barrier leg cuff 64 may have a first barrier cuff elastic material 72 adjacent to the proximal portion 68 and a second barrier cuff elastic material 73 adjacent to the distal portion 70 of the barrier leg cuff 64. The distal portion 70 of the barrier leg cuff 64 may be joined to the backsheet 60 adjacent to the longitudinal side edge 48. The portion of the barrier leg cuff 64 along the folding line 66 and the proximal portion 68 may be free from attachment to any portion of the center chassis 38 in the crotch region 30 such that the barrier leg cuff 64 stands up toward the wearer's body. The transverse end 74 of the barrier leg cuff 64 may be joined to the topsheet 58 at or adjacent the longitudinally opposing ends of the leg cuff by an attachment means which may be any known means such as an adhesive, heat bond, pressure bond or the like.

The liquid pervious topsheet 58 may be positioned adjacent the body-facing surface of the absorbent core 62 and may be joined thereto and/or to the backsheet 60 by any attachment means known in the art. The liquid impervious backsheet 60 is generally that portion of the absorbent article 20 positioned adjacent the garment-facing surface of the absorbent core 62 and prevents the exudates absorbed and contained therein from soiling articles that may contact the absorbent article 20. The absorbent core is positioned between the topsheet 58 and the backsheet 60 and absorbs and retains liquids such as urine and other certain body exudates.

The topsheet 58, the backsheet 60 and the absorbent core may be manufactured any known materials. Suitable topsheet materials may include porous foams; reticulated foams; apertured plastic films; or woven or nonwoven webs of natural fibers (e.g., wood or cotton fibers), synthetic fibers (e.g., polyester or polypropylene fibers), or a combination of natural and synthetic fibers. Suitable backsheet materials may include breathable materials that permit vapors to escape from the diaper while still preventing exudates from passing through the backsheet. Suitable backsheet materials may include, films, microporous breathable films, monolithic breathable films, nonwovens or combinations thereof.

A suitable absorbent core for use in the absorbent article 20 may comprise any absorbent material which is generally compressible, conformable, non-irritating to the wearer's skin, and capable of absorbing and retaining liquids such as urine and other certain body exudates. In addition, the configuration and construction of the absorbent core may also be varied (e.g., the absorbent core(s) or other absorbent structure(s) may have varying caliper zones, hydrophilic gradient(s), a superabsorbent gradient(s), or lower average density and lower average basis weight acquisition zones; or may comprise one or more layers or structures). In some embodiments, the absorbent core may comprise a fluid acquisition component, a fluid distribution component, and a fluid storage component. An example of a suitable absorbent core having a fluid acquisition component, a fluid distribution component, and a fluid storage component is described in US6,590,136.

The outer cover layer 42 may be disposed on the outer surface 22 of the absorbent article 20 and cover the crotch panel 56 of the absorbent center chassis 38. The outer cover layer 42 may extend into and cover the front waist panel 52 and the back waist panel 54 of the center chassis 38. The outer cover layer may form a portion of the backsheet and/or the center chassis. The outer cover layer 42 may be directly joined to and cover a portion or all of the liquid impervious backsheet 60 of the center chassis 38. The central panel 80 of the front and back belt 84, 86 may be joined to the front waist panel 52 and the back waist panel 54 of the center chassis 38 through the outer cover layer 42. The outer cover layer 42 may be disposed between the front and back belt 84, 86 and the liquid impervious backsheet 60 of the center chassis 38. In one embodiment shown in Figures 2 and 4c, the outer cover layer 42 is coextensive with the liquid impervious backsheet 60. The leg elastic material 140 is disposed so as to extend generally longitudinally along the longitudinal side edge 48 of the center chassis 38. The leg elastic material 140 may be disposed at least in the crotch region 30 of the absorbent article 20 or may be disposed along the entirety of the longitudinal side edge 48.

The outer cover layer 42 may comprise a material separate from the material of the inner layer 83 and the outer layer 82 constituting the belt 40. The outer cover layer 42 may comprise one or more layers of materials. The outer cover layer 42 may comprise any known materials and may comprise materials used for the front and back belt 84, 86 as explained above. The outer cover layer 42 may comprise a single layer of nonwoven web of synthetic fibers. The outer cover layer 42 may comprise a single layer of hydrophobic, non-stretchable nonwoven material. The outer cover layer may comprise a fibrous web comprising a film, a foam, a nonwoven, a woven material or the like and/or combinations thereof such as a laminate of a film and a nonwoven. Fibrous materials can be made from animal fibers, plant fibers, mineral fibers, synthetic fibers, etc. Fibrous materials can include short fibers, long fibers, continuous fibers, fibers of varying lengths or cross-sectional geometries, or combinations of any of these. In some cases, a fibrous material can include another material, can be joined to another material, or can be incorporated into another material. Fibrous materials can take many forms, such as fabrics, textiles, and composites. Examples of fabrics include fibrous textiles (woven or knitted fabrics), felts, nonwovens, papers, and others. Examples of fibrous composites include composite materials with polymeric fibers, carbon fibers, glass fibers, and metal fibers, to name a few. Throughout the present disclosure, nonwoven materials are used to describe and illustrate various embodiments. However, it is contemplated that embodiments of the present disclosure are not limited to nonwoven materials, but can be similarly applied to a wide variety of fibrous materials, such as those described above, as will be understood by one of skill in the art.

As an example, methods of the present disclosure can be used to create realistic models of fibrous nonwoven materials. The term "nonwoven material" refers to a sheet-like structure (e.g. web) of fibers (sometimes referred to as filaments) that are interlaid in a non-uniform, irregular, or random manner. A nonwoven material can be a single layer structure or a multiple layer structure. A nonwoven material can also be joined to another material, such as a film, to form a laminate.

A nonwoven material can be made from various natural and/or synthetic materials. Exemplary natural materials include cellulosic fibers, such as cotton, jute, pulp, and the like; and also can include reprocessed cellulosic fibers like rayon or viscose. Natural fibers for a nonwoven material can be prepared using various processes such as carding, etc.

Exemplary synthetic materials include but are not limited to synthetic thermoplastic polymers that are known to form fibers, which include, but are not limited to, polyolefins, e.g., polyethylene, polypropylene, polybutylene and the like; polyamides, e.g., nylon 6, nylon 6/6, nylon 10, nylon 12 and the like; polyesters, e.g., polyethylene terephthalate, polybutylene terephthalate, polylactic acid and the like; polycarbonate; polystyrene; thermoplastic elastomers; vinyl polymers; polyurethane; and blends and copolymers thereof.

The belt 40 comprises a front belt 84 and a rear belt 86 (hereinafter may be referred to as "front and back belt" 84, 86) and has a ring-like configuration by permanently or refastenably connecting the front belt 84 and the back belt 86 at the seams 32.

The belt 40 may be ring-like and elastic. The ring-like elastic belt 40 extends transversely about at least a portion of the waist opening 36 of the absorbent article 20 and acts to dynamically create fitment forces and to distribute the forces dynamically generated during wear. Applicants have found that improved fit can be created by controlling one or both of the distance, spacing, and/or the pre-strain of the elastomeric material in relation to each other and to the openings for the body. This may occur by choosing different materials throughout the belt 40 that exhibit desired properties. The different materials are combined at specific distances to create a belt 40 that acts to dynamically create fitment forces. This improved fit translates into reduced sagging and or gapping problems around the waist opening.

The front and back belt 84, 86 may comprise any known materials. Suitable material for the front and back belt 84, 86 can be manufactured from a wide range of materials such as plastic films; apertured plastic films; woven or nonwoven webs of natural materials (e.g., wood or cotton fibers), synthetic fibers (e.g., polyolefins, polyamides, polyester, polyethylene, or polypropylene fibers), or a combination of natural and/or synthetic fibers; or coated woven or nonwoven webs. The belt may comprise a nonwoven web of synthetic fibers. The belt may comprise a stretchable nonwoven. The belt may comprise an inner hydrophobic, non-stretchable nonwoven material and an outer hydrophobic, non-stretchable nonwoven material.

The belt 40 may comprise a first elastic section 102 and a second elastic section 104 located in the front belt 84. The belt 40 may comprise a third elastic section 106 and a fourth elastic section 108 located in the back belt 86. The first elastic section 102 and the fourth elastic section 108 are adjacent to the waist opening 36. The second elastic section 104 and the third elastic section 106 are adjacent to the leg openings 34. The first elastic section 102 may comprise of 20 percent to 80 percent, 25 percent, 40 percent, 50 percent, 60 percent, 70 percent of the longitudinal direction of the front belt 84. The second elastic section 104 may comprise of 20 percent to 80 percent, 30 percent, 40 percent, 50 percent, 60 percent, or 70 percent of the longitudinal direction of the front belt 84. The third elastic section 106 may comprise of 20 percent to 80 percent, 25 percent, 40 percent, 50 percent, 60 percent, 70 percent of the longitudinal direction of the back belt 86. The fourth elastic section 108 may comprise of 20 percent to 80 percent, 25 percent, 40 percent, 50 percent, 60 percent, 70 percent of the longitudinal direction of the back belt 86.

The belt 40 may comprise a front border between the first elastic section 102 and a second elastic section 104, and the front border may be located within 5mm, 10mm, 20mm, 30mm, 40mm, 50mm from the front edge of the absorbent core. The belt 40 may comprise a back border between the third elastic section 106 and a fourth elastic section 108, and the back border may be located within 5mm, 10mm, 20mm, 30mm 40mm, 50mm from the back edge of the absorbent core.

The belt 40 may comprise a first force zone 110, a second force zone 112, a third force zone 114, a fourth force zone 116, a fifth force zone 118, and a sixth force zone 120 located in the front belt 84. The first force zone 110, second force zone 112, and third force zone 114 may be located in the first elastic section 102. The fourth force zone 116, fifth force zone 118, and sixth force zone 120 may be located in the second elastic section 104. The first force zone 110, second force zone 112, third force zone 114, fourth force zone 116, fifth force zone 118 and sixth force zone 120 may comprise a transverse force of 0 to 10N/zone. The force in a zone may also change between the center of the belt 40 and the left and right longitudinally extending side edges 48.

The first force zone 110 is disposed adjacent to the waist opening 36. The sixth force zone 120 is disposed adjacent to the leg opening 34. The first force zone 110, second force zone 112, and at least part of the third force zone 114 are located within upper two thirds of the front belt width, toward the waist opening along the longitudinal axis. At least part of the fourth force zone 116, fifth force zone 118, and sixth force zone 120 are located within lower two third of the front belt width, toward the leg opening 34.

The belt 40 may comprise a seventh force zone 122, a eight force zone 124, a ninth force zone 126, a tenth force zone 128, a eleventh force zone 130, and a twelfth force zone 132 located in the back belt 86. The seventh force zone 122, eight force zone 124, and ninth force zone 126 may be located in the third elastic section 106. The tenth force zone 128, eleventh force zone 130, and twelfth force zone 132 may be located in the fourth elastic section 108. The seventh force zone 122, eighth force zone 124, ninth force zone 126, tenth force zone 128, eleventh force zone 130 and twelfth force zone 132 may comprise a transverse force of 0 to 10N/zone. The seventh force zone 122 is adjacent to the leg opening 34. The twelfth force zone 132 is adjacent to the waist opening 36. The seventh force zone 122, eighth force zone 124, and at least part of the ninth force zone 126 are located within lower two third of the back belt width, toward the leg opening 34. Force zones may be equally distanced throughout the belt along the longitudinal axis in the front and back belts 84, 86. Force zones may also be unequally distanced throughout the belt along the longitudinal axis in the front and back belts 84, 86. Force zones may have varying width and length. Force zones may be continuous or discontinuous, as for example, when disrupted by the center chassis 38 and/or absorbent core.

Many of the embodiments disclosed herein describe the graphics in terms of the placement of the graphic in the structure of the article. It should be understood that the intent of the present invention is to create structures that have an equal opacity of the layers between the first graphic and the outer surface and the second graphic and the outer surface such that the appearance of the first graphic and second graphic are uniform, as well as an equal gloss, color, and web uniformity. This may be done by choosing an equal number of nonwoven layers covering the first and second graphics, equal total basis weight of the summed layers, choice of different nonwovens such as one with higher basis weight comprised of higher diameter fibers and one with lower basis weight comprised of lower diameter fibers, or other such nonwoven material selections that provide similar resultant optical properties known to one of ordinary skill in the art.

Alternatively, parameters, such as the CMC color scale (a modification of the CIE LAB color scale) and/or optical density measurements can be used to describe articles with uniform graphics. In some embodiments, a first graphic may be printed on one or more layers of the front elastomeric belt and a second graphic may be printed on one or more layers of the center chassis, wherein the color difference between the first graphic and the second graphic, $\Delta E_1$, as determined by CMC may be less than about 5.0, and wherein the difference in optical density between the first graphic and the second graphic may be less than about 0.3. In some embodiments, the first graphic may be printed on a viewable surface of the front elastomeric belt, on an outer surface of a layer in the front elastomeric belt, on an inner surface of a layer in the front elastomeric belt, on the inner surface of the first belt substrate, or on the outer surface of the second belt substrate. In other embodiments, the outer layer of the front elastomeric belt may be a nonwoven. In some embodiments, the second graphic may be printed on the inner surface of a layer of the center chassis. In other embodiments, the first belt substrate comprises at least one nonwoven layer and the second belt substrate comprises at least one nonwoven layer, and elastic elements may be disposed between the nonwoven layers of the first and second belt substrates. In still other embodiments, a third graphic may be printed on one or more layers of the rear elastomeric belt, wherein the color difference between the third graphic and the second graphic, $\Delta E_2$ as determined by the CMC color scale, may be less than about 5.0, and wherein the difference in optical density between the third graphic and the second graphic may be less than about 0.3. In some embodiments, the color difference between any two graphics, as determined by CMC, may be less than about 5.0, less than about 4.0, less than about 3.0, less than about 2.5, or less than about 2.0, and the difference in optical density between any two graphics may be less than about 0.3, less than about 0.25, or less than about 0.2.

In some embodiments, a first graphic may have a first intensity (as measured by, for example, CMC color scale or optical density), and a second graphic may have a second intensity that is different than the first intensity, wherein the first and second graphics are covered with materials having different opacity such that the difference in opacity from each graphic to the outer surface of the article offsets the difference in print intensity, providing a uniform graphic appearance. That is, in some embodiments, two graphics may not necessarily have the same color or density, yet when viewed from the outer surface of the absorbent article, the graphics may appear uniform.

The obtained absorbent article comprises graphics disposed on or spanning multiple viewable absorbent article components while creating a uniform appearance.

Test Methods

Color:

The L*a*b* color space has traditionally been used for the color difference task. Color differences are expressed as Delta E, a value based on the Euclidian distance (the shortest line in three dimensions) between the coordinates of the reference and sample (Note: the word "Delta" is often shown as its Greek symbol, $\Delta$, a small triangle). For the L*a*b* space this difference is called Delta $E^*_{ab}$.

While this color difference formula is the one used most often, it was found that the computed color difference did not precisely correspond to the perceived color difference for all possible sets of compared colors. Efforts in making this color difference even more uniform have first brought the CIE94 and CMC color difference formulas. Another color difference formula is DeltaE2000 (CIEDE2000). These three color difference formulas, CIE94, CMC, and CIEDE20000 are all based on L*a*b* data, to which they add correction and weighing factors. In all of these, the goal is that a color difference Delta E of 1 corresponds to a barely noticeable difference by 50% of the persons comparing the two patches.

Other numbers, labeled as Delta L*, Delta C*, Delta H*, and Delta h*, correspond to the difference in lightness (L*, perceived luminance), Chroma (C*, describing color saturation), hue (H*), and hue angle (h*, hue expressed as an angle between 0 and 360 degrees) between a sample and a standard. Yet another measure is Delta D* the difference in optical density.

The CMC test method is described in AATCC Test Method 173, "CMC: Calculation of Small Color Differences for Acceptability". Color differences calculated using the CMC method are believed to correlate better with visual assessment than color differences calculated using other instrumental systems. The CMC method has two parameters, lightness (l) and chroma (c). The CMC equations are based on an ellipsoidal space with semi-axis lengths of SL, SC, and SH. The CMC ratio l:c influences the shape of the ellipsoid. The c (chroma) is usually smaller than the l (lightness) because humans perceive smaller shifts in chroma than in lightness. The l:c ratio is typically set at 2:1 for most applications. An h, hue, value may be added to this ratio, but h is always 1, so it is not included. The SL, SC, and SH are calculated based on the CIELCh values. They are used to set the base size and shape of the ellipsoid. The SL is multiplied by l and SC is multiplied by c to set the shape. A commercial factor may be set to change the size of the ellipsoid.

The $\Delta E_{cmc}$ is the total color difference value in this system. This number is useful as a single number indicator of the difference between a sample and a standard. Due to the method of calculation, the $\Delta E_{cmc}$ value allows the evaluation of the acceptability of a color match without regard to the color of the standard (e.g., two reds that have a $\Delta E_{cmc}$ of 0.5 have the same amount of visual color difference as two blues that have a $\Delta E_{cmc}$ of 0.5). As a result, a single $\Delta E_{cmc}$ limit value may be set to be used in evaluating the color matches of all graphics produced.

The measurement of optical density is still the most frequently used method for checking and controlling ink transfer in the printing process. Densitometry is especially suitable when producing four-color process images using cyan, magenta, yellow and black ink. Measurement of dot area in a tone scale is designed to check the mechanical ability of a process to reproduce a given dot structure and is therefore used in pre-press to control film and plate dot, as well as in the pressroom to measure the print process. Regardless of whether densities are measured in the film, on the printing plate or in color bars on a printed sheet, the result is always a single value. This value corresponds to the relationship between the light that strikes the sample and the light that is reflected off the sample. In all cases, density measures light fractions as values of gray. Actual color tones cannot be measured by means of densitometry.

Density is also a unitless value. Density is a function of the percentage of light reflected. Density = $\log_{10} 1/R$. Where R = Reflectance, Ink film thickness is approximately proportional to optical density. Keep in mind that a printing press naturally varies, and a typical tight tolerance for density is $\pm$ 0.05 D.

Definitions

CIELAB Color, the tristimulus color scale based on CIE 1976 standard, containing a lightness (L*), amber (a*) and blue (b*) term; the total color difference is calculated from the CIE 1976 L* a* b* opponent-color scales, and is denoted as $\Delta E^*$

CIE Chroma, the attribute of color used to indicate the degree of departure of the color from a gray of the same lightness; the chroma difference is calculated by using the CIE 1976 a* b* opponent-color scales, denoted as $\Delta C^*$

CIE Hue, the attribute of color perception by means of which a color is judged to be red, orange, yellow, green, blue, purple or intermediate color; the hue difference is calculated by using the CIE 1976 a* b* opponent-color scales, denoted as $\Delta H^*$

Equipment

Reflectance Spectrophotometer ...... 45°/0°
Recommended....GretagMacbeth SpectroEye Spectrophotometer Divtech Equipment P.O. Box 58468 Cincinnati, Ohio 45258 Phone: 513-941-0483
E-mail: GWeckenbrock@Divtechequipment.com
Webpage: www.GretagMacbeth.com
Scissors........Convenient type
Tissue..........Convenient type, without embossing, lotion, UV or fluorescence brighteners.
White Standard Cardboard ......... #PG2000 Provided by Sun Chemical - Vivitek Division:
1701 Westinghouse Blvd, Charlotte, NC 28273, USA. Phone: (704)-587-8381

Facilities

The samples and instrument should be kept in an area free of high humidity and corrosive vapors, and the samples should be protected from contamination by dirt or lint.

Instrument Setup

| Physical filter | No |
|---|---|
| White base: | Abs |
| Illuminant: | C |
| Observer Angle: | 2 degrees |
| Density standard: | ANSI T |
| Formula: | Delta E* (CMC) |
| Note: | |

- Ensure that the spectrophotometer is set to calculate L*a*b* and not standard Hunter Lab values.
- Calibrate the spectrophotometer according to manufacturer's instructions or SOP before beginning testing.
- Recommended Instrument: GretagMacbeth SprectroEye Spectrophotometer / Densitometer.
- Any instrument other than GretagMacbeth SpectroEye must be verified via Lab Co-op.

Test Procedure

1. Select a sample region for analysis.
2. Carefully place 1 ply or multiple layers as defined in specification of the sample over the PG2000 White Standard Cardboard. Position the spectrophotometers measurement aperture such that the sample is centered in the sample region. NOTE: The colored sample region must be larger than the sample measurement aperture to avoid erroneous measurements.
3. Read and record L* a* b* or Density values.

The color difference, or $\Delta E$, between a sample color $L^*_2 a^*_2 b^*_2$ and a reference color $L_1 a_1 b_1$ is:

$$\Delta E = \sqrt{\left(\Delta L / l\, S_L\right)^2 + \left(\Delta C / c\, S_C\right)^2 + \left(\Delta H / S_H\right)^2}$$

Reporting

1. L*, a*, b* values are reported to the nearest 0.1 units $\Delta C^*$, $\Delta E^*$, and $\Delta H^*$ (CMC).
2. Density: $\Delta C^*$, $\Delta E^*$, and $\Delta H^*$ values are to be reported to the nearest 0.01 units.

Opacity

Purpose

Opacity is a measure of the capacity of a material to obscure the background behind it. Opacity measurements are sensitive to material thickness and degree of pigmentation (e.g., % TiO2). Normally a value for opacity is determined by dividing the reflectance obtained with a black backing (RB) for the material, by the reflectance obtained for the same material with a white backing (WB). This is called the "contrast ratio (C R/) method".

That is:

$$\% \text{ Opacity} = CR \times 100 = RB \times 100 RW$$

If the Hunter colorimeter is set to the X, Y, Z color scale, opacity may be defined as:

% Opacity = Y reading over black plate x 100 Y reading over white plate

Apparatus

Reflectance

Hunter Labscan XE, Hunter D25DP9000, or equivalent Spectrophotometer 45°/0° Hunter Lab Headquarters, 11491 Sunset Hills Road, RestonVA 20190-5280 Tel: 703-471-6870 Fax: 703-471-4237 http://hunterlab.com/

Standard Plates

A set of two plates consisting of white, black available from the Colorimeter manufacturer.

Tissue

Soft absorbent tissue without embossing or lotion, such as Puffs for cleaning the Standard Plates.

Cutter

Any convenient type

Facilities

Conditioned Room

An effect of normal laboratory temperature and humidity ranges upon sample color is negligible, thus samples need not be conditioned prior to determining opacity. The samples and instrument should be kept in an area free of high humidity and corrosive vapors, however, and the samples should be protected from contamination by dirt or lint.

Sample Preparation

Generally, 10.16cm by 10.16 cm portions of sample are cut for analysis from a sample.
Samples as small as 5mm X 5mm can be measured by the device however, larger samples up to 44mm will enable measurements with less variability due to non-uniformity of the sample itself. Most samples are easily cut using a cutting die a hydraulic cutter such as an Alfa cutter. Scissors or paper cutter may be used; however, care must be taken that this does not destroy product needed for other analyses.
Select sample free from creases, wrinkles, tears, and other obvious defects for testing.
Always stack and fold the sample in such a way that the outer surface of the product as it is converted will be the top surface of the sample "stack" directly under the instrument sample port, unless instructions for a particular material indicates to the contrary.
If sheet orientation exists, make the sample so that the MD is identical for all samples.
Select a portion of sample for analysis. Cut single 1-ply samples, 10.16cm * 10.16 cm, or largest size feasible from the product available as long as it is at least 5mm X 5mm with the machine direction perpendicular and/or parallel to the cut edges, from each sample to be tested using an appropriate cutting device.

Equipment Preparation

Calibrate the spectrophotometer using standard black and white tiles supplied with the instrument according to manufacturer's instructions or SOP before beginning any testing.
Set the color scale to XYZ, the Observer to 10 ° and the illuminant to D65.

Test Procedure

Place the white standard plate with the Sample according to manufacturer's instructions into the spectrophotometer.
Without contaminating the test area of the sample, place it on top of the white standard plate which should be placed so that the machine direction is parallel to a line splitting the standard plate in half from left to right. The sample should also be placed (with the embossed side if embossed) of the poly facing the light source.
Record the "Y" reading to the nearest 0.1 unit.
Repeat steps 2 through 4 above using the black standard plate in place of the white standard plate.

Calculation Reporting

$$\% \text{ Opacity} = [\text{``Y''} \text{ (black plate)}] \times 100[\text{``Y''} \text{ (white plate)}]$$

Report Opacity (%) to the nearest 0.1 unit.

Basis Weight

This Method is technically identical with compendial method(s) ASTM D 756, ISO 536 & ERT-40.3-90.

Gloss

This method is technically identical with methods ASTM D2457-97, with the exceptions that testing is to be run at a 45 degree angle and with 10-ply layers for film materials.

## Claims

**1.**   A pull-on disposable absorbent article (20) comprising a front elastomeric belt (84) disposed in a first waist region, a rear elastomeric belt (86) disposed in a second waist region, and a center chassis (38) disposed in a crotch region (30), said center chassis overlapping and extending between the front elastomeric belt and the rear elastomeric belt;

said article comprising an outer surface and an inner surface; said absorbent article further comprising a topsheet (58), a backsheet (60), an absorbent core (62), and wherein the backsheet is a film and nonwoven laminate, wherein the nonwoven of the laminate forms an outer cover (42) having an outer surface and an inner surface; wherein the front elastomeric belt and the rear elastomeric belt comprise a first belt substrate (82) and the front elastomeric belt and the rear elastomeric belt comprise a second belt substrate (83);
wherein the first belt substrate is disposed outward from the second belt substrate;
wherein said first belt substrate comprises at least one nonwoven layer, each nonwoven layer having an outer surface and an inner surface;
wherein said second belt substrate comprises at least one nonwoven layer, each nonwoven layer having an outer surface and an inner surface;
wherein the center chassis comprises at least one nonwoven layer and at least one film layer, each nonwoven layer having an outer surface and an inner surface and each film layer having an outer surface and an inner surface;
wherein the first belt substrate is in two discontinuous portions, a first portion in the front elastomeric belt (82a, 83a) and a second portion in the rear elastomeric belt (82b, 83b);
wherein the second belt substrate is in two discontinuous portions, a first portion in the front elastomeric belt and a second portion in the rear elastomeric belt (82b, 83b);
said article comprising a first graphic (90) and a second graphic (91);
**characterized in that**:

wherein the first graphic is printed on the outer surface of a nonwoven layer of the first and second portions of the first belt substrate;
wherein either the second graphic is printed on the inner surface of the outer cover nonwoven that is in the crotch region, or the second graphic is printed on the outer surface of the film layer of the backsheet that is in the crotch region; and
wherein the number of nonwoven layers between any first graphic and the outer surface of the article is the same as the number of nonwoven layers between any second graphic and the outer surface of the article.

## Patentansprüche

**1.**   Einweg-Absorptionsartikel (20) zum Anziehen, umfassend ein vorderseitiges Elastomerband (84), das in einem ersten Taillenbereich angeordnet ist, ein hinterseitiges Elastomerband (86), das in einem zweiten Taillenbereich angeordnet ist, und eine zentrale Grundeinheit (38), die in einem Schrittbereich (30) angeordnet ist, wobei die zentrale Grundeinheit das vorderseitige Elastomerband und das hinterseitige Elastomerband überlappt und sich dazwischen erstreckt;

der Artikel umfassend eine Außenoberfläche und eine Innenoberfläche; der Absorptionsartikel ferner umfassend eine Oberschicht (58), eine Unterschicht (60), einen Absorptionskern (62) und wobei die Unterschicht eine Folie und Vlieslaminat ist, wobei das Vlies des Laminats einen Außenmantel (42) bildet, der eine Außenoberfläche und eine Innenoberfläche aufweist;

wobei das vorderseitige Elastomerband und das hinterseitige Elastomerband ein erstes Bandsubstrat (82) umfassen und das vorderseitige Elastomerband und das hinterseitige Elastomerband ein zweites Bandsubstrat (83) umfassen;

wobei das erste Bandsubstrat außerhalb des zweiten Bandsubstrats angeordnet ist;

wobei das erste Bandsubstrat mindestens eine Vliesschicht umfasst, wobei jede Vliesschicht eine Außenoberfläche und eine Innenoberfläche aufweist;

wobei das zweite Bandsubstrat mindestens eine Vliesschicht umfasst, wobei jede Vliesschicht eine Außenoberfläche und eine Innenoberfläche aufweist;

wobei die zentrale Grundeinheit mindestens eine Vliesschicht und mindestens eine Folienschicht umfasst, wobei jede Vliesschicht eine Außenoberfläche und eine Innenoberfläche aufweist und jede Folienschicht eine Außenoberfläche und eine Innenoberfläche aufweist;

wobei sich das erste Bandsubstrat in zwei ununterbrochenen Abschnitten befindet, ein erster Abschnitt in dem vorderseitigen Elastomerband (82a, 83a) und ein zweiter Abschnitt in dem hinterseitigen Elastomerband (82b, 83b);

wobei sich das zweite Bandsubstrat in zwei ununterbrochenen Abschnitten befindet, ein erster Abschnitt in dem vorderseitigen Elastomerband und ein zweiter Abschnitt in dem hinterseitigen Elastomerband (82b, 83b);

der Artikel umfassend eine erste Grafik (90) und eine zweite Grafik (91);

**dadurch gekennzeichnet, dass:**

wobei die erste Grafik auf die Außenoberfläche einer Vliesschicht des ersten und des zweiten Abschnitts des ersten Bandsubstrats gedruckt ist;

wobei entweder die zweite Grafik auf die Innenoberfläche des Außenmantelvlieses gedruckt ist, die sich in dem Schrittbereich befindet, oder die zweite Grafik auf die Außenoberfläche der Folienschicht der Unterschicht gedruckt ist, die sich in dem Schrittbereich befindet; und

wobei die Anzahl von Vliesschichten zwischen einer beliebigen ersten Grafik und der Außenoberfläche des Artikels gleich der Anzahl von Vliesschichten zwischen einer beliebigen zweiten Grafik und der Außenoberfläche des Artikels ist.

## Revendications

1. Article absorbant jetable à enfiler (20) comprenant une ceinture élastomère avant (84) disposée dans une première région de taille, une ceinture élastomère arrière (86) disposée dans une seconde région de taille, et une armature centrale (38) disposée dans une région d'entrejambe (30), ladite armature centrale chevauchant et s'étendant entre la ceinture élastomère avant et la ceinture élastomère arrière ;

ledit article comprenant une surface externe et une surface interne ; ledit article absorbant comprenant en outre une feuille de couverture (58), une feuille de fond (60), une âme absorbante (62), et dans lequel la feuille de fond est un film et un stratifié non tissé, dans lequel le nontissé du stratifié forme une couverture externe (42) ayant une surface externe et une surface interne ;

dans lequel la ceinture élastomère avant et la ceinture élastomère arrière comprennent un premier substrat de ceinture (82) et la ceinture élastomère avant et la ceinture élastomère arrière comprennent un second substrat de ceinture (83) ;

dans lequel le premier substrat de ceinture est disposé vers l'extérieur du second substrat de ceinture ;

dans lequel ledit premier substrat de ceinture comprend au moins une couche non tissée, chaque couche non tissée ayant une surface externe et une surface interne ;

dans lequel ledit second substrat de ceinture comprend au moins une couche non tissée, chaque couche non tissée ayant une surface externe et une surface interne ;

dans lequel l'armature centrale comprend au moins une couche non tissée et au moins une couche de film, chaque couche non tissée ayant une surface externe et une surface interne et chaque couche de film ayant une surface externe et une surface interne ;

dans lequel le premier substrat de ceinture est en deux parties discontinues, une première partie dans la ceinture élastomère avant (82a, 83a) et une seconde partie dans la ceinture élastomère arrière (82b, 83b) ;

dans lequel le second substrat de ceinture est en deux parties discontinues, une première partie dans la ceinture élastomère avant et une seconde partie dans la ceinture élastomère arrière (82b, 83b) ;

ledit article comprenant un premier graphisme (90) et un second graphisme (91) ;

**caractérisé en ce que** :

dans lequel le premier graphisme est imprimé sur la surface externe d'une couche non tissée des première et seconde parties du premier substrat de ceinture ;

dans lequel soit le second graphisme est imprimé sur la surface interne du nontissé de couverture externe qui est dans la région d'entrejambe, soit le second graphisme est imprimé sur la surface externe de la couche de film de la feuille de fond qui est dans la région d'entrejambe ; et

dans lequel le nombre de couches non tissées entre l'un quelconque premier graphisme et la surface externe de l'article est identique au nombre de couches non tissées entre l'un quelconque second graphisme et la surface externe de l'article.

Fig. 1

Fig. 2

Fig. 3

Fig. 4a

Fig. 4b

134

83

90

26

90

82

84

30

20

58

91

62

60

38

28

82

86

90

90

83

138

Fig. 4c

Fig. 4d

Fig. 4c

Fig. 4f

EP 2 849 709 B1

Fig. 4g

28

Fig. 5

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2011090000 A1
- US 20060264858 A1
- US 5518801 A, Chappell
- US 5366782 A, Curro
- US 6590136 B